# EUROPÄISCHE PATENTSCHRIFT

(11) **EP 2 074 960 B1**
(45) Veröffentlichungstag und Bekanntmachung des Hinweises auf die Patenterteilung: **30.10.2013**
(21) Anmeldenummer: 08021883.7
(22) Anmeldetag: 17.12.2008
(51) Int. Cl.: A61F 2/06

(54) **Gewebte textile Gefässprothese**
Webbed textile stent
Prothèse vasculaire textile tissulaire

(30) Priorität: 17.12.2007 DE 102007063265
(43) Veröffentlichungstag der Anmeldung: 01.07.2009
(73) Patentinhaber: Aesculap AG, 78532 Tuttlingen (DE)
(72) Erfinder: Goldmann, Helmut, 34119 Kassel (DE); Merckle, Christof, 68199 Mannheim (DE)
(74) Vertreter: Patentanwälte Ruff, Wilhelm, Beier, Dauster & Partner

(56) Entgegenhaltungen:
- WO-A-92/03107
- WO-A-99/40875
- US-A- 2 924 250
- US-A- 3 095 017
- US-A- 6 136 022
- US-A1- 2002 058 992
- US-A1- 2005 240 261

## Beschreibung

Die Erfindung betrifft eine gewebte textile Gefäßprothese mit einem rohrförmigen Stamm und mindestens einer Verzweigung und je einem Scheitel zwischen abgehenden Rohrästen geringeren Durchmessers. Gewebte rohrförmige Gefäßprothesen mit einer Verzweigung sind aus der EP 0 108 171 B1 und der EP 0 910 310 B1 (entspricht der DE 697 28 268 T2) bekannt. Solche Verzweigungen werden auch als Gabelungen oder Bifurkation oder Trifurkation bezeichnet. Bei Gefäßprothesen mit Verzweigung besteht das Problem, dass im Gabelungsbereich bzw. im Zwickel der Verzweigung Undichtigkeiten auftreten. Diese Undichtigkeiten sind darauf zurückzuführen, dass beim Übergang vom großen in die kleinen Durchmesser im Zwickelbereich eine zusätzliche Fläche vorhanden ist, die bei gegebener Fadenzahl nicht in der gewünschten Dichtigkeit bedeckt werden kann. Dieses Problem ist in der DE 697 28 268 T2 ausführlich beschrieben. Zur Lösung des Problems wird dort vorgeschlagen, schlagen, eine graduelle Änderung in der Anzahl von Kettfäden vorzunehmen. Insbesondere wird vorgeschlagen, im Übergangsbereich Teile von Kettfäden, die für die eine Abzweigung bestimmt sind, noch mit Schussfäden der anderen Abzweigung zu verbinden und umgekehrt, so dass ein allmählicher Übergang vom Stamm in die Abzweigungen erfolgt. Weiterhin wird beim Übergang vom Stamm in die Abzweigungen die Zahl der Kettfäden erhöht.

Die US 2005/0240261 A1 offenbart eine bifurkierte Gefäßprothese, deren Zwickelbereich gewebt und deren übrigen Bereiche gewirkt vorliegen. Aus der WO 99/40875 A1 ist eine gewebte Gefäßprothese mit einem sich konisch verjüngenden Zwischenabschnitt und zwei sich daran anschließenden Seitenabschnitten unterschiedlichen Durchmessers bekannt. Der Zwischenabschnitt weist Kettfäden auf, welche in Richtung des Seitenabschnitts mit dem kleineren Durchmesser zunehmend dichter aneinander gelegt sind.

Die Erfindung stellt sich die Aufgabe, eine bessere Lösung für das Dichtigkeitsproblem zu schaffen.

Dieses Problem wird ausgehend von der oben genannten Prothese dadurch gelöst, dass die Prothesenwandung im Bereich des Scheitels dichter gewebt ist als eine Originalbindung in anderen Bereichen. Vorzugsweise ist die Prothesenwanderung nur im Bereich des Scheitels dichter gewebt. Eine Änderung der Zahl der Kettfäden und eine Überschneidung der Schussfäden der abgehenden Äste ist vorzugsweise nicht vorgesehen.

Gewebte Prothesen sind normalerweise dichter bzw. können dichter eingestellt werden als gewirkte Prothesen. Wird beim Weben mit gleichbleibender Fadenzahl der Kettfäden gearbeitet, wie es bei der Erfindung der Fall ist, dann besteht im sogenannten Zwickelbereich der Verzweigung durch die zusätzliche Oberfläche eine unerwünschte Undichtigkeit. Diese wird gemäß der Erfindung dadurch beseitigt, dass in diesem Bereich das Gewebe dichter gewebt wird als in den übrigen Bereichen. Eine Unterbrechung des Webvorgangs oder eine nachträgliche Bearbeitung entfällt somit. Die Verzweigung ist eine solche, bei der die Kettfäden vom dickeren Stamm in die abzweigenden Äste weiterlaufen. In der Regel handelt es sich um eine Bifurkation. Erfindungsgemäß kann auch eine Trifurkation oder eine höhere Verzweigung vorgesehen sein. Die Herstellung der Prothese ist in einfacher Weise möglich.

Die Aufspaltung der Schussfäden am Übergang vom Stamm in die Äste erfolgt vorzugsweise von einem Schuss zum anderen. Die Gefäßprothese kann plissiert oder unplissiert sein. Sie kann auch mit üblichen Imprägnierungen oder Beschichtungen versehen sein.

Der dichtere Bereich ist mindestens teilweise durch eine dichtere Bindungsart als die der Originalbindung gewebt. Die gesamte Gefäßprothese in einer solchen dichteren Bindungsart zu weben, wäre auf der anderen Seite weniger günstig, weil es sich beim Krümmungsbereich ja um eine gegenüber den anderen Bereichen erhöhte Fläche pro Kettfadenzahl handelt. Außerdem sind Flottungen und/oder Velourbildungen in den anderen Bereichen erwünscht, um ein gutes Einwachsen von Bindegewebe zu ermöglichen.

Eine andere oder zusätzliche Art, den gekrümmten Bereich dichter zu bekommen, besteht darin, dass die Schussfäden im Bindungsbereich dichter aneinander liegen, insbesondere dichter gepackt sind als in den anderen Bereichen. Jede dieser Maßnahmen reicht an sich aus, eine gewünschte Dichtigkeit zu erzielen. Die Kombination beider Maßnahmen ist jedoch in besonderer Weise vorteilhaft. Die Kettfadenzahl ist vorzugsweise konstant. Es kommen insbesondere keine neuen Kettfäden über die Länge der Prothese hinzu, noch werden solche abgenommen.

Der dichter gewebte Bereich erstreckt sich gemäß einer weiteren Ausführungsform der Erfindung je nach dem Durchmesser der Prothese über mindestens 30 Kettfäden, vorzugsweise über 30 bis 130 Kettfäden. Mit besonderem Vorteil erstreckt sich der verdichtete Bereich über mindestens einen Rapport der Originalbindung der anderen Bereiche. Erstreckt sich beispielsweise der Rapport über 32 Kettfäden, so umfasst der verdichtete Bereich vorzugsweise 32, 64, 96 oder 128 Kettfäden.

In ähnlicher Weise erstreckt sich der dichter gewebte Bereich vorzugsweise über mindestens 10 Schussfäden, insbesondere über 10 bis 50 Schussfäden, vorzugsweise über 10 bis 15 Schussfäden. Beträgt der Rapport der Schussfäden beispielsweise 16 Fäden, dann erstreckt sich der Bereich vorzugsweise über 16, 32 oder 48 Schussfäden. Besonders gute Ergebnisse werden erhalten, wenn sich der dichter gewebte Bereich über mehr als die Hälfte der Kettfäden der Prothese erstreckt.

Wie oben bereits erwähnt, ist die Originalbindung der Gefäßprothese normalerweise dazu eingerichtet, dass ein gutes Einwachsen des Bindegewebes ermöglicht wird. Zumindest auf der Außenseite der Gefäßprothese sind deshalb gewisse Auflockerungen wie Velourschlingen, Texturierungen und Flottungen bevorzugt. Als Gewebebindungen sind deshalb für die übrigen Bereiche Bindungsarten vorgesehen, die solche Varianten ermöglichen. Geeignete Bindungsarten sind beispielsweise Gewebe mit Veloursbindungen, die sich insbesondere dadurch auszeichnen, dass die Innenseite der gewebten Prothese glatt ausgebildet ist und die Außenseite durch flottierende texturierte Kettfäden das Einwachsen des Bindegewebes begünstigt. Ähnliche Effekte können auch kettseitige Körper- und Atlasbindungen erreichen.

Für den dichter gewebten Bereich sind bei einer bevorzugten Ausführungsform der Erfindung Bindungsarten vorgesehen, die keine oder nur wenige Flottungen besitzen und bei denen texturierte Fäden, die normalerweise weniger dicht sind als glatte Fäden, nur über geringfügige Längen offenliegen. Als dichte Bindung eignet sich in erster Linie eine Leinwandbindung. Eine Leinwandbindung mit 1 über 1, 1 unter 1 ist die dichteste Gewebebindung. Weitere Bindungsarten, die für den dichter gewebten Bereich geeignet sind, sind beispielsweise verschiedene Leinwandabwandlungen wie die Panama- und Ripsbindung sowie kleinraportiger Köper (z.B. K 2/1 oder K 1/2 in S oder Z-Grad).

Kommt noch eine Verdichtung der Schussfäden hinzu, bei der die Schussfäden dichter liegen, vorzugsweise um 5 bis 20 %, d.h. sich in entsprechender Weise über weniger Kettenlänge erstrecken, dann wird die gewünschte Dichtigkeit problemlos erreicht. Eine mechanische Verdichtung kann insbesondere durch einen stärkeren Anschlag der Schussfäden erreicht werden. Der Prozentsatz der maximalen Verdichtung bzw. Kompaktierung hängt von der Gewebedichte der Originalbindung und von der Dichte bzw. dem Garndurchmesser der Schussfäden ab. Ferner kann durch die Verwendung von Schrumpfgarnen im dichter gewebten Bereich eine erhöhte Dichtigkeit des Gewebes durch nachträglichen Schrumpf erzielt werden. Beim Schrumpfen nehmen die Schrumpfgarne in der Dicke zu, wodurch die Verdichtung erreicht wird. Zur Vermeidung einer Tallienbildung durch den Schrumpf der Schussfäden können die Kettfäden im dichteren Bereich beim Weben in dem Maße gespreizt werden, z.B. mit Hilfe einer V-Schiene, in dem der Schrumpf den Faden verkürzt. Außerhalb des dichteren Bereiches werden vorzugsweise nicht oder nur wenig schrumpffähige Schussgarne verwendet.

Die für die Gefäßprothese verwendeten Fäden sind vorzugsweise multifile Fäden. Es können sowohl glatte Fäden als auch texturierte Fäden vorgesehen sein. Bevorzugt weist die Gewebebindung der Gefäßprothese eine Kette aus glatten und texturierten Fäden, insbesondere Garnen auf. Bei einer besonders bevorzugten Ausführungsform wechseln sich glatte und texturierte Fäden in der Kette ab, bevorzugt im Verhältnis 1:1. Bei den Schussfäden sind ausschließlich glatte Fäden bevorzugt. Es überwiegen somit die glatten Fäden. Durch geeignete Bindungsart kann aber erreicht werden, dass texturierte Fäden bevorzugt auf der Außenfläche der Prothese liegen, was für ein Einwachsen günstig ist.

Gemäß der Erfindung ist somit weder ein nachträgliches Vernähen im Scheitelbereich noch die Verwendung zusätzlicher Kettfäden beim Webvorgang vorgesehen. Trotzdem wird die gewünschte Dichtigkeit im Zwickel der Verzweigung erreicht.

Weitere Merkmale der Erfindung ergeben sich aus der nachfolgenden Beschreibung von bevorzugten Ausführungsformen in Verbindung mit den Unteransprüchen und der Zeichnung. Hierbei können die einzelnen Merkmale jeweils für sich alleine oder zu mehreren in Kombination miteinander verwirklicht sein.

In der Zeichnung zeigen:
- Figur 1:: eine erfindungsgemäße Gefäßprothese,
- Figur 2:: ein Bindungsschema für eine Ausführungsform nach der Er- findung, reduziert um die auf der hinteren Prothesenseite (Schlauchrückwand) bindenden Kett- und Schussfäden und
- Figur 3:: ein Bindungsschema für die Ausführungsform nach Figur 2 inklusive der auf der hinteren Prothesenseite bindenden Kett- und Schussfäden.

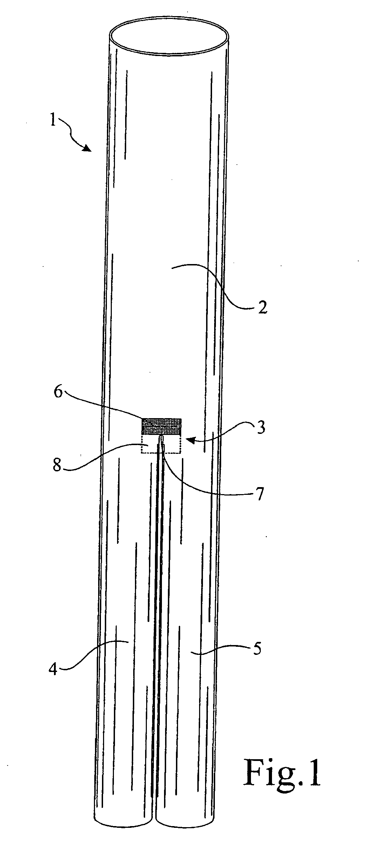

Bei der in der Zeichnung in Figur 1 dargestellten Ausführungsform der Erfindung ist eine erfindungsgemäße gewebte Gefäßprothese 1 mit einer Bifurkation dargestellt. Die Gefäßprothese 1 weist einen rohrförmigen Stamm 2 auf, der eine Verzweigung bzw. Bifurkation 3 besitzt und an der Verzweigungsstelle in zwei in Verlängerung des Stammes weiter gehende Äste bzw. Verzweigungen 4 und 5 übergeht. Die Durchmesser der ebenfalls rohrförmigen Äste entsprechen etwa dem halben Durchmesser des Stamms.

Das Gewebe der Gefäßprothese weist in Längsrichtung verlaufende Kettfäden und quer dazu umlaufende Schussfäden auf. Die Kettfäden erstrecken sich über die gesamte Länge der Gefäßprothese und teilen sich in der Bifurkation hälftig auf. Anstelle des einen umlaufenden Schussfadens im Stamm ist ab der Bifurkation für jeden Ast ein getrennter Schussfaden vorgesehen. Die Gefäßprothese wird im Prinzip in an sich bekannter Weise hergestellt, so wie dies auch in der DE 697 28 268 T2 beschrieben ist. Es wird zunächst ein doppeltes Flachgewebe hergestellt, wobei eine untere Lage und eine obere Lage beim Weben an den Rändern miteinander verbunden werden. Die Prothese kann in an sich bekannnter Weise plissiert sein. Sie kann, falls erwünscht, eine Imprägnierung aus einem resorbierbaren Material besitzen. Hergestellt werden kann die Gefäßprothese als sogenanntes Endlosgewebe, bei dem sich ein Stamm 2 in zwei Äste 4 und 5 aufteilt, die sich dann nach einer vorgesehenen Strecke wieder zu einem neuen Stamm vereinigen, der sich dann nach einer bestimmten Länge wieder in zwei Abzweigungen aufteilt. Durch Herausschneiden geeigneter Längsabschnitte kann jeweils die Prothese mit Bifurkation erhalten werden.

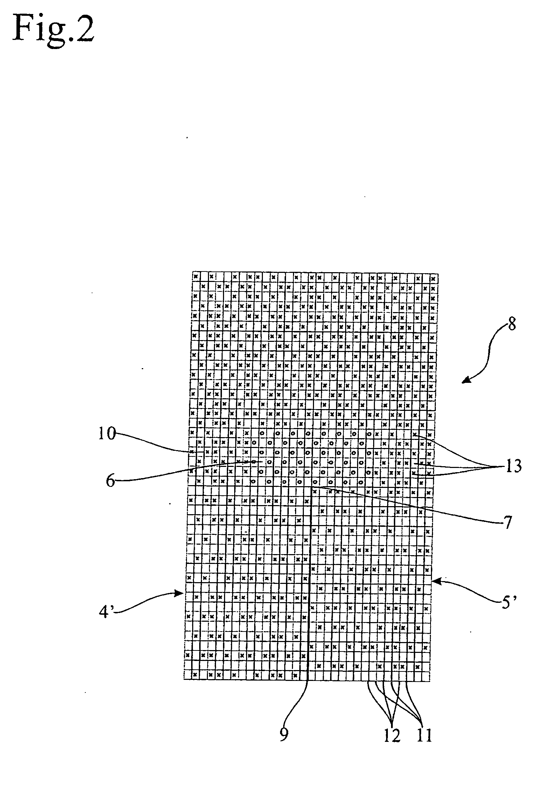

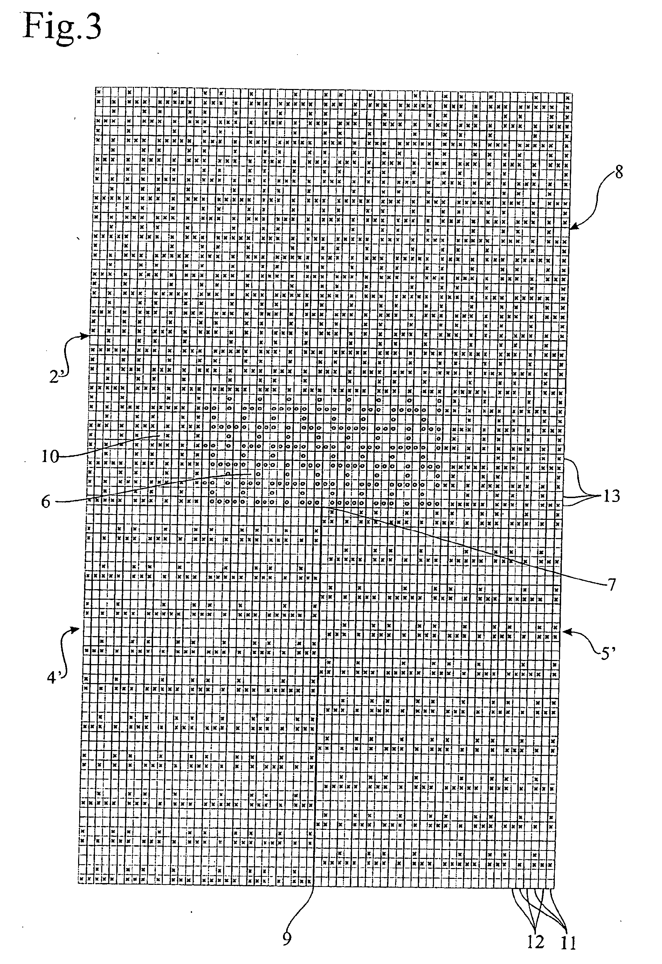

Im Bereich der Bifurkation befindet sich im Stamm 2 kurz vor der Aufteilung in die beiden Äste 4 und 5 ein Bereich 6, in dem das Gewebe dichter ist als in den übrigen Bezirken. Dadurch wird eine Verdichtung des Gewebes im Zwickel 7 der Bifurkation erhalten, wodurch die sonst hier auftretende größere Porosität verhindert wird. Ein eingerahmtes Feld 8, das den dichteren Bereich 7 einschließt, ist in den Figuren 2 und 3 dargestellt und dort näher erläutert.

Figur 2 zeigt ein Bindungsschema der Gewebebindung im Bereich 8 von Figur 1 für eine Ausführungsform der Erfindung. Die untere Hälfte zeigt zwei schlauchförmige Abschnitte 4' und 5', die voneinander getrennt sind, wie dies durch die Trennlinie 9 angedeutet ist. Die Abschnitte 4' und 5' sind bereits Teile der Äste 4 und 5. Im mittleren Bereich vereinigen sich die beiden schlauchförmigen Abschnitte 4' und 5' und gehen in einen Schlauchabschnitt 2' mit entsprechend doppeltem Durchmesser über, der Teil des Stammes 2 ist. An der Übergangsstelle befindet sich die Bifurkationsstelle 7.

Die Originalbindung des Gewebes nach Figur 2 und 3 ist eine um Velourfäden erweiterte Leinwandbindung, wobei die Velourfäden an der Außenseite liegen. Sie ist an sich schon relativ dicht. Sie erstreckt sich vom Schlauchabschnitt 2 großen Durchmessers und über die Schläuche 4 und 5 kleineren Durchmessers sowie an den Außenrändern 10 des Übergangsbereiches in die Bifurkation. Die Bindungsart im dichter gewebten Bereich 6 an der Übergangsstelle ist eine reine Leinwandbindung 1 über 1, 1 unter 1 (1/1). Diese besonders dichte Gewebebindung ermöglicht die gewünschte Abdichtung an der Bifurkationsstelle 7 von Figur 1.

Als Kettfäden sind glatte multifile Fäden 11 vorgesehen, die sich mit texturierten Fäden 12 abwechseln. Als Schussfäden 13 sind ausschließlich glatte multifile Fäden vorgesehen. Geeignete glatte Fäden haben die Charakterisierung 100f 80Z 240. Geeignete texturierte Fäden haben die Kennzeichnung 100f 80Z 140.

Figur 3 zeigt dieselbe Ausführungsform wie Figur 2, wobei jedoch auch die Schlauchrückwand in das Bindungsschema miteinbezogen ist. Deshalb ist das Bindungsschema in Länge und Breite auseinander gezogen. Im dichter gewebten Bereich 6 können die Schussfäden zusätzlich in Kettfadenrichtung kompaktiert sein, wodurch die Dichtigkeit zusätzlich erhöht wird. Alternativ oder zusätzlich können in diesem Bereich auch Schrumpfgarne als Schussfäden eingesetzt werden.

## Patentansprüche

1. Gewebte textile Gefäßprothese (1) mit einem rohrförmigen Stamm und mindestens einer Verzweigung und je einem Scheitel zwischen abgehenden Rohrästen geringen Durchmessers, **dadurch gekennzeichnet, dass** die Prothesenwandung im Bereich des Scheitels (7) dichter gewebt ist als in anderen Bereichen mit Originalwebbindung und der dichter gewebte Bereich (7) eine Bindungsart aufweist, die dichter ist als die Originalbindung.

2. Gewebte textile Gefäßprothese nach Anspruch 1, **dadurch gekennzeichnet, dass** sich Schussfäden des Stammes im Scheitel (7) von einem Schuss zum anderen in voneinander unabhängige Schussfäden der Abzweigungen (4 und 5) aufteilen.

3. Gewebte textile Gefäßprothese nach Anspruch 1 oder 2, **dadurch gekennzeichnet, dass** der dichter gewebte Bereich (7) ein dichteres Aneinanderliegen der Schussfäden (14) aufweist als in den anderen Bereichen.

4. Gewebte textile Gefäßprothese nach einem der vorhergehenden Ansprüche, **dadurch gekennzeichnet, dass** sich der dichtere Bereich (7) über mindestens 30 Kettfäden (12, 13), vorzugsweise über 30 bis 130 Kettfäden erstreckt (32,64,128).

5. Gewebte textile Gefäßprothese nach einem der vorhergehenden Ansprüche, **dadurch gekennzeichnet, dass** sich der dichter gewebte Bereich (7) über mindestens 10 Schüsse (14), vorzugsweise über 10 bis 15 Schüsse erstreckt (16,32 oder 48).

6. Gewebte textile Gefäßprothese nach einem der vorhergehenden Ansprüche, **dadurch gekennzeichnet, dass** sich der dichtere Bereich (7) über mindestens einen Rapport der Originalbindung erstreckt.

7. Gewebte textile Gefäßprothese nach einem der vorhergehenden Ansprüche, **dadurch gekennzeichnet, dass** die Originalbindung eine mit Velourfäden modifizierte Leinwandbindung ist.

8. Gewebte textile Gefäßprothese nach einem der vorhergehenden Ansprüche, **dadurch gekennzeichnet, dass** die Bindung im dichteren Bereich (7) eine Leinwandbindung ist.

9. Gewebte textile Gefäßprothese nach einem der vorhergehenden Ansprüche, **dadurch gekennzeichnet, dass** die Bindung im dichteren Bereich (7) eine Abwandlung einer Leinwandbindung ist.

10. Gewebte textile Gefäßprothese nach einem der vorhergehenden Ansprüche, **dadurch gekennzeichnet, dass** die Gewebebindung eine Kette aus glatten und texturierten Fäden aufweist, wobei sich die glatten (11) und texturierten (12) Fäden in der Kette abwechseln.

11. Gewebte textile Gefäßprothese nach einem der vorhergehenden Ansprüche, **dadurch gekennzeichnet, dass** Schussfäden (14) ausschließlich glatte Fäden sind.

12. Gewebte textile Gefäßprothese nach einem der vorhergehenden Ansprüche, **dadurch gekennzeichnet, dass** die Schussfäden zumindest außerhalb des dichter gewebten Bereiches geschrumpfte Schrumpfgarne sind.

13. Gewebte textile Gefäßprothese nach einem der vorhergehenden Ansprüche, **dadurch gekennzeichnet, dass** der dichter gewebte Bereich (8) frei von zusätzlichen Kettfäden ist.

14. Gewebte textile Gefäßprothese nach einem der vorhergehenden Ansprüche, **dadurch gekennzeichnet, dass** die Anzahl der Kettfäden über die ganze Länge der Prothese konstant ist.

## Claims

1. A woven textile vascular prosthesis (1) having a tubular trunk and at least one fork, each with an apex located between outgoing tubular branches having a small diameter, **characterized in that** the wall of the prosthesis in the region of the apex (7) is woven more densely than in the other zones having the original weave construction, and **in that** the more densely woven zone (7) has a construction that is denser than the original construction.

2. The woven textile vascular prosthesis according to claim 1, **characterized in that** the weft yarns of the trunk in the apex (7) split up from one weft yam to another into independent weft yarns of the forks (4 and 5).

3. The woven textile vascular prosthesis according to claim 1 or 2, **characterized in that** the weft yarns (14) lie closer to each other in the more densely woven zone (7) than in the other zones.

4. The woven textile vascular prosthesis according to any one of the preceding claims, **characterized in that** the denser zone (7) extends over at least 30 warp yarns (12, 13), preferably over 30 to 130 warp yarns (32, 64, 128).

5. The woven textile vascular prosthesis according to any one of the preceding claims, **characterized in that** the denser zone (7) extends over at least 10 weft yarns (14), preferably over 10 to 15 weft yarns (16, 32 or 48).

6. The woven textile vascular prosthesis according to any one of the preceding claims, **characterized in that** the denser zone (7) extends over at least at least one repeat of the original construction.

7. The woven textile vascular prosthesis according to any one of the preceding claims, **characterized in that** the original construction is a plain weave modified with velour yarns.

8. The woven textile vascular prosthesis according to any one of the preceding claims, **characterized in that** the construction in the denser zone (7) is a plain weave.

9. The woven textile vascular prosthesis according to any one of the preceding claims, **characterized in that** the construction in the denser zone (7) is a variation of a plain weave.

10. The woven textile vascular prosthesis according to any one of the preceding claims, **characterized in that** the woven construction has a warp comprising untextured and textured yarns, with untextured (11) and textured (12) yarns alternating in the warp.

11. The woven textile vascular prosthesis according to any one of the preceding claims, **characterized in that** the weft yarns (14) are exclusively untextured yarns.

12. The woven textile vascular prosthesis according to any one of the preceding claims, **characterized in that** the weft yarns, at least outside the more densely woven zone, are shrink yarns that have shrunk.

13. The woven textile vascular prosthesis according to any one of the preceding claims, **characterized in that** the more densely woven zone (8) is free of additional warp yarns.

14. The woven textile vascular prosthesis according to any one of the preceding claims, **characterized in that** the number of warp yarns is constant over the entire length of the prosthesis.

## Revendications

1. Prothèse vasculaire textile tissée (1) comportant un tronc tubulaire et au moins une ramification et un sommet entre chacun des rameaux tubulaires de faible diamètre qui s'étendent, **caractérisée en ce que** dans la zone du sommet (7) la paroi de la prothèse est à tissage plus dense que dans les autres zones à armure d'origine et la zone à tissage plus dense (7) présente un type d'armure qui est plus dense que l'armure d'origine.

2. Prothèse vasculaire textile tissée selon la revendication 1, **caractérisée en ce que** dans le sommet (7) les fils de trame du tronc se divisent d'une duite à l'autre en fils de trame des ramifications (4 et 5) indépendants les uns des autres.

3. Prothèse vasculaire textile tissée selon la revendication 1 ou 2, **caractérisée en ce que** la zone à tissage plus dense (7) présente un assemblage plus dense des fils de trame (14) que dans les autres zones.

4. Prothèse vasculaire textile tissée selon l'une quelconque des revendications précédentes, **caractérisée en ce que** la zone plus dense (7) s'étend sur au moins 30 fils de chaîne (12, 13), de préférence sur 30 à 130 fils de chaîne (32, 64, 128).

5. Prothèse vasculaire textile tissée selon l'une quelconque des revendications précédentes, **caractérisée en ce que** la zone à tissage plus dense (7) s'étend sur au moins 10 duites (14), de préférence sur 10 à 15 duites (16, 32 ou 48).

6. Prothèse vasculaire textile tissée selon l'une quelconque des revendications précédentes, **caractérisée en ce que** la zone plus dense (7) s'étend sur au moins un rapport de l'armure d'origine.

7. Prothèse vasculaire textile tissée selon l'une quelconque des revendications précédentes, **caractérisée en ce que** l'armure d'origine est une armure toile modifiée avec des fils velours.

8. Prothèse vasculaire textile tissée selon l'une quelconque des revendications précédentes, **caractérisée en ce que** l'armure dans la zone plus dense (7) est une armure toile.

9. Prothèse vasculaire textile tissée selon l'une quelconque des revendications précédentes, **caractérisée en ce que** l'armure dans la zone plus dense (7) est une modification d'une armure toile.

10. Prothèse vasculaire textile tissée selon l'une quelconque des revendications précédentes, **caractérisée en ce que** l'armure du tissu présente une chaîne de fils lisses et de fils texturés, les fils lisses (11) et les fils texturés (12) alternant dans la chaîne.

11. Prothèse vasculaire textile tissée selon l'une quelconque des revendications précédentes, **caractérisée en ce que** les fils de trame (14) sont exclusivement des fils lisses.

12. Prothèse vasculaire textile tissée selon l'une quelconque des revendications précédentes, **caractérisée en ce qu'**au moins en dehors de la zone à tissage plus dense les fils de trame sont des fils rétractables rétractés.

13. Prothèse vasculaire textile tissée selon l'une quelconque des revendications précédentes, **caractérisée en ce que** la zone à tissage plus dense (8) est exempte de fils de chaîne supplémentaires.

14. Prothèse vasculaire textile tissée selon l'une quelconque des revendications précédentes, **caractérisée en ce que** le nombre des fils de chaîne est constant sur toute la longueur de la prothèse.
